# EUROPÄISCHE PATENTSCHRIFT

(11) **EP 3 752 208 B1**
(45) Veröffentlichungstag und Bekanntmachung des Hinweises auf die Patenterteilung: **18.10.2023**
(21) Anmeldenummer: 19706431.4
(22) Anmeldetag: 13.02.2019
(51) Int. Cl.: A61L 2/08, A61K 35/12

(54) **VERFAHREN ZUR ERHÖHUNG DER SICHERHEIT UND VERRINGERUNG DER ANZAHL MITOTISCHER ZELLEN VON GEWERBEPRÄPARATEN FÜR DIE KLINISCHE ANWENDUNG**
METHOD FOR INCREASING THE SAFETY AND REDUCTION OF MITOTICAL CELLS OF TISSUE PREPARATIONS FOR CLINICAL APPLICATION
PROCÉDÉ POUR ACCROÎTRE LA SÉCURITÉ ET REDUCTION DES CELLULES MITOTIQUES DE PRÉPARATIONS DE TISSUS POUR L'APPLICATION CLINIQUE

(30) Priorität: 14.02.2018 DE 102018202268
(43) Veröffentlichungstag der Anmeldung: 23.12.2020
(73) Patentinhaber: BioMed Invest UG (haftungsbeschränkt), 37073 Göttingen (DE)
(72) Erfinder: ZIMMERMANN, Wolfram-Hubertus, 37073 Göttingen (DE); TIBURCY, Malte, 37083 Göttingen (DE)
(74) Vertreter: Grund, Martin
(86) Internationale Anmeldenummer: PCT/EP2019/053520
(87) Internationale Veröffentlichungsnummer: WO 2019/158568

(56) Entgegenhaltungen:
- WO-A1-2004/022077
- WO-A1-2004/022077
- WO-A2-2008/011524
- WO-A2-2008/011524
- MALTE TIBURCY ET AL: "Modeling myocardial growth and hypertrophy in engineered heart muscle", TRENDS IN CARDIOVASCULAR MEDICINE, 1. August 2013 (2013-08-01), XP055083983, ISSN: 1050-1738, DOI: 10.1016/j.tcm.2013.05.003
- DAI WANGDE ET AL: "Myocardial regeneration by embryonic stem cell transplantation: present and future trends", EXPERT REVIEW OF CARDIOVASCULAR THE, EXPERT REVIEWS LTD, GB, Bd. 4, Nr. 3, 1. Mai 2006 (2006-05-01), Seiten 375-383, XP009130777, ISSN: 1744-8344, DOI: 10.1586/14779072.4.3.375
- JOHN P CHUTE ET AL: "Ex vivo culture rescues hematopoietic stem cells with long-term repopulating capacity following harvest from lethally irradiated mice", EXPERIMENTAL HEMATOLOGY, Bd. 32, Nr. 3, 1. März 2004 (2004-03-01), Seiten 308-317, XP055005295, ISSN: 0301-472X, DOI: 10.1016/j.exphem.2003.12.002
- Malte Tiburcy ET AL: "Modeling myocardial growth and hypertrophy in engineered heart muscle", Trends in Cardiovascular Medicine, 1 August 2013 (2013-08-01), XP055083983, ISSN: 1050-1738, DOI: 10.1016/j.tcm.2013.05.003
- DAI WANGDE ET AL: "Myocardial regeneration by embryonic stem cell transplantation: present and future trends", EXPERT REVIEW OF CARDIOVASCULAR THERAPY, EXPERT REVIEWS LTD, GB, vol. 4, no. 3, 1 May 2006 (2006-05-01), pages 375-383, XP009130777, ISSN: 1744-8344, DOI: 10.1586/14779072.4.3.375
- John P Chute ET AL: "Ex vivo culture rescues hematopoietic stem cells with long-term repopulating capacity following harvest from lethally irradiated mice", Experimental hematology, vol. 32, no. 3, 1 March 2004 (2004-03-01), pages 308-317, XP055005295, ISSN: 0301-472X, DOI: 10.1016/j.exphem.2003.12.002

## Beschreibung

### Technisches Gebiet der Erfindung

Die Erfindung entstammt dem Gebiet der Behandlung zellulärer Gewebeprodukte ("tissue engineered products"(TEP)).

### Hintergrund der Erfindung

Das Ziel der regenerativen Medizin ist der Ersatz von erkrankten Körperzellen oder kompletten Geweben/Organen durch im Labor hergestellte zelluläre Produkte (zum Beispiel *tissue engineered products"* (TEP)). Als wichtigste Quelle für "Ersatzzellen" haben sich in den letzten Jahren pluripotente Stammzellen, also embryonale oder induzierte pluripotente Stammzellen, herauskristallisiert. Diese Zellen lassen sich unbegrenzt expandieren und können sich zu jeder Zelle des menschlichen Körpers differenzieren. Bei unkontrollierter Differenzierung können allerdings Tumore (Teratome) entstehen, die Gewebe aller drei Keimblätter enthalten. Teratome können in seltenen Fällen spontan im Menschen entstehen. Insbesondere aber kann nach Implantation von pluripotenten Zellen ein Teratom entstehen (Cao *et al.,* 2006). Bereits 1×10⁴-1×10⁵ pluripotente Zellen waren in einem Rattenmodell fähig, ein Teratom zu bilden (Lee *et al.,* 2009). Bei Zelltherapien, die potentiell Milliarden von Zellen, die aus pluripotenten Zellen abgeleitet sind, enthält, sind minimale Kontaminationen mit residualen pluripotenten Zellen nicht auszuschließen und selbst mit sensitivsten Methoden (wie qPCR) nicht zu detektieren.

Dies ist ein kritischer Sicherheitsaspekt bei Anwendung jedes zellulären Produkts aus pluripotenten Stammzellen. Es muss sichergestellt sein, dass keine teilungsfähigen pluripotenten Zellen mehr enthalten sind. Potentielle Lösungsvorschläge für dieses Problem sind positive bzw. negative Selektion von therapeutisch nutzbaren Zellen bzw. von tumorigenen pluripotenten Restzellen (z.B. über Antibiotikaresistenzen, Suizidgene, fluoreszierende Reportergene, Oberflächenmarker-basierte Selektion). Allerdings sind diese Verfahren zum Teil aufwendig und es ist unklar, ob sie aus regulatorischer Sicht überhaupt einsetzbar wären. Daher besteht der Bedarf nach einem zuverlässigen, leicht durchzuführenden Verfahren zur mitotischen Inaktivierung von zellulären Produkten aus pluripotenten Stammzellen.

Um dieses Problem zu lösen, verwendet die vorliegende Erfindung Bestrahlung als einen nicht-genetischen und leicht durchführbaren Ansatz der mitotischen Inaktivierung. Bestrahlung mit dem Ziel der mitotischen Inaktivierung ist eine Standardprozedur bei Blutprodukten, um teilungsfähige Leukozyten zu hemmen und damit eine Graft-versus-Host-Reaktion (GvHD) zu verhindern. Typischerweise wird dazu eine Strahlendosis von 25-30 Gy eingesetzt (Treleaven *et al.,* 2011). Bei der Gefahr eines malignen Wachstums von im Blut enthaltenen Zellen wurden 50 Gy zur sicheren mitotischen Inaktivierung vorgeschlagen (Hansen *et al.,* 1999). Allerdings kann Bestrahlung auch die eigentlich erwünschte biologische Aktivität der Zellen beeinträchtigen. Im Falle von Blutprodukten wie Erythrozyten und Thrombozytenkonzentraten stellt dies kein Problem dar, weil die Zellen ohnehin kernlos sind und erwünschte Effekte wie Sauerstofftransport, Blutstillung etc. nicht beeinträchtigt werden. Hingegen besteht bei der Bestrahlung von zellulären Produkten, deren Funktion auf lebenden Zellen beruht, immer die Gefahr eines bestrahlungsbedingten Funktionsverlustes. Zum Beispiel führte die Behandlung von embryonalen Stammzellen mit Strahlendosen von 25-100 Gy zu einem vollständigen Verlust der Zell-Vitalität nach spätestens 7 Tagen (Burt *et al.,* 2012). Auch resultierte die Behandlung von *engineered heart muscle* (EHM) mit der Strahlendosis von 60 Gy, gefolgt von sofortiger Transplantation, zum vollständigen Funktionsverlust des zellulären Produktes (Riegler *et al.,* 2015).

Um zellulären Funktionsverlust zu vermeiden, verwenden *in vivo*-Bestrahlungen sehr geringe Strahlendosen von 1,8-2,0 Gy pro Bestrahlung (siehe z.B. Emami, 2013). Solch geringe Strahlendosen könnten jedoch nicht die sichere Deaktivierung der mitotischen Zellen in TEPs sicherstellen.

Die vorliegende Erfindung zeigt erstmalig, dass Bestrahlung ein geeignetes Verfahren zur mitotischen Inaktivierung bei TEPs, insbesondere muskulären TEPs, ist, ohne dass es zum Funktionsverlust kommt. Dafür werden zwei alternative Ansätze gewählt:
Zum einen kann die Bestrahlung mit exakt definierten Strahlendosen durchgeführt werden, sodass proliferative Zellen ausgeschaltet und zugleich der Funktionsverlust von Anfang an verhindert oder minimiert wird.

In einem anderen Ansatz kann die Bestrahlung mit Strahlendosen durchgeführt werden, die proliferative Zellen ausschalten, aber auch die biologische Funktion des TEP zumindest temporär beeinträchtigen. Diese Beeinträchtigung kann durch folgende Regeneration des TEP in Zellkultur ausgeglichen werden, sodass man nach Bestrahlung und Regeneration ein TEP ohne proliferative Zellen, aber mit biologischer Funktion erhält.

Die Erfindung garantiert somit eine hohe Sicherheit (kein Tumorwachstum) eines TEP bei erhaltener biologischer Aktivität (z.B. Kontraktion, Krafterzeugung bei muskulären TEP).

WO 2008 011 524 A1 offenbart ein Verfahren zur Erhöhung der Sicherheit bei einem Stammzellprodukt, wobei das Verfahren die Bestrahlung des Zellprodukts mit einer Strahlendosis zwischen 10 und 50 Gy umfasst.

### Zusammenfassung der Erfindung

Die Erfindung wird durch Anspruch 1 definiert.

In manchen Ausführungsformen sind dabei nach der Bestrahlung weniger als 10% mitotische Zellen im TEP vorhanden. In manchen bevorzugten Ausführungsformen sind dabei nach der Bestrahlung sogar weniger als 1% mitotische Zellen im TEP vorhanden.

In bevorzugten Ausführungsformen bleibt eine biologische Funktion des TEP nach der Bestrahlung im Wesentlichen erhalten.

In manchen Ausführungsformen wird die Bestrahlung mit Röntgenstrahlen durchgeführt.

In manchen Ausführungsformen erfolgt die Bestrahlung mit einer Strahlungsdosis von 15-45 Gy, bevorzugt von 20-40 Gy, noch bevorzugter von 25-35 Gy und insbesondere von 30 Gy. In anderen Ausführungsformen bietet die Vorliegende Erfindung ein Verfahren zur Erhöhung der Sicherheit bei einem *tissue engineered product* (TEP), wobei das Verfahren die Bestrahlung des TEP mit einer Strahlendosis zwischen 10 und 100 Gy und die Regeneration des TEP in Zellkultur umfasst.

In manchen Ausführungsformen sind dabei nach der Bestrahlung weniger als 10% mitotische Zellen im TEP vorhanden. In manchen bevorzugten Ausführungsformen sind dabei nach der Bestrahlung sogar weniger als 1% mitotische Zellen im TEP vorhanden.

In bevorzugten Ausführungsformen bleibt eine biologische Funktion des TEP nach der Regeneration im Wesentlichen erhalten, verglichen mit dem Zustand vor der Bestrahlung. In manchen Ausführungsformen ist eine biologische Funktion des TEP nach der Regeneration sogar verbessert, verglichen mit dem Zustand vor der Bestrahlung.

In manchen Ausführungsformen wird die Bestrahlung mit Röntgenstrahlen durchgeführt.

In manchen Ausführungsformen erfolgt die Bestrahlung mit einer Strahlungsdosis von 10-50 Gy, bevorzugt von 20-40 Gy, noch bevorzugter von 25-35 Gy und insbesondere von 30 Gy. In manchen Ausführungsformen erfolgt die Regeneration für mindestens einen Tag, für 2 bis 30 Tage oder für 10 bis 20 Tage.

Bei bevorzugten Verfahren der vorliegenden Erfindung ist das TEP ein muskuläres TEP (TEM), insbesondere ein *engineered heart muscle* (EHM) oder ein *engineered skeletal muscle* (ESM). Die im Wesentlichen erhaltene biologische Funktion des TEP ist in bevorzugten Ausführungsformen Kontraktion (Kontraktionskraft, Kontraktilität).

Bevorzugt ist das TEP geeignet zur Verwendung bei Transplantation und bei Arzneimittelentwicklung ist.

### Kurze Beschreibung der Abbildungen

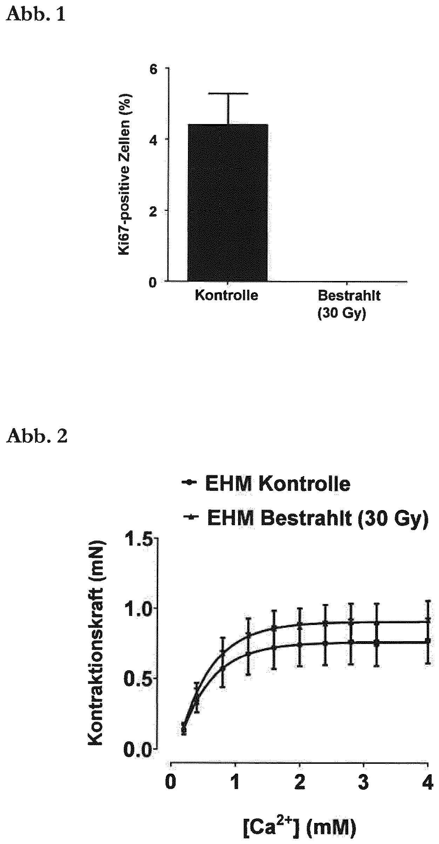

**Abbildung 1****. Bestrahlung hemmt die Zellzyklusaktivtät in menschlichem Herzmuskel.** Messung der Zellzyklusmarker-Ki67-positiven Zellen in humanem *engineered heart muscle* (EHM) mittels Durchflusszytometrie. Vergleich von unbestrahlter Kontrolle und mit 30 Gy bestrahltem Gewebe; n=4 Kontrolle und n=10 Bestrahlt.

**Abbildung 2****. Bestrahlung hat keinen Einfluss auf die biologische Aktivität von humanem Herzmuskel.** Kontraktionskraft von *engineered heart muscle* (EHM) mit zunehmender extrazellulärer Kalziumkonzentration. Die EHM wurden an Tag 3 der Kultur mit 30 Gy bestrahlt und dann für weitere 4 Wochen kultiviert; n=5 Kontrolle und n=13 Bestrahlt.

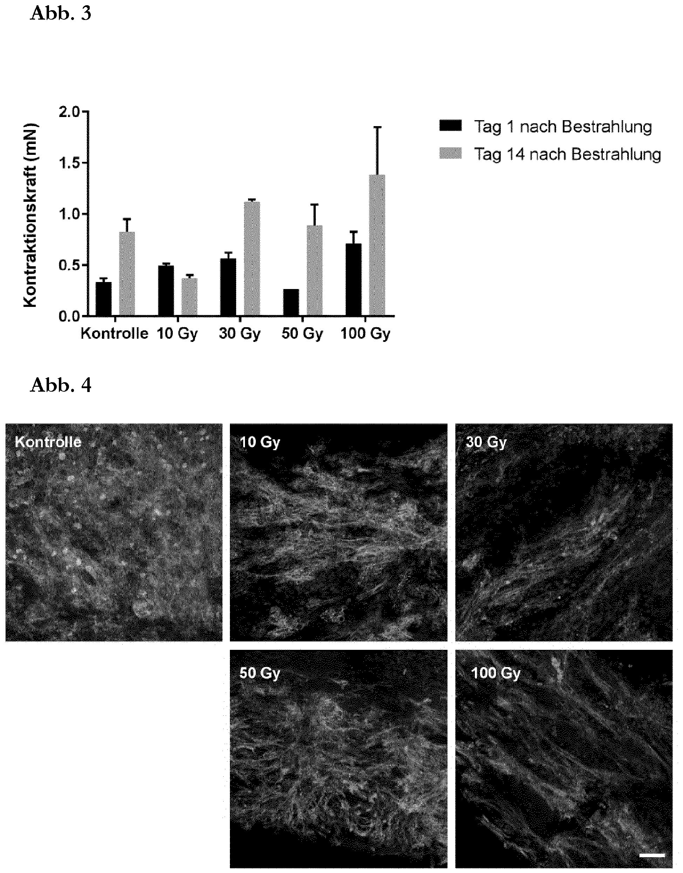

**Abbildung 3****. Dosis-Wirkung der Bestrahlung auf die Kontraktionskraft von humanem Herzmuskel.** Vergleich von akuten Effekten der Bestrahlung (Tag 1 nach Bestrahlung) und chronischen Effekten (Tag 14 nach Bestrahlung). Kontraktionskraft (Ordinate) von humanen EHM nach Bestrahlung mit jeweiliger Dosis (Abszisse). n=3 pro Gruppe außer 50 Gy Tag 1 (n=2).

**Abbildung 4****. Dosis-Wirkung der Bestrahlung auf die Zellzyklusaktivität von humanem Herzmuskel.** Färbung der Zellzyklusmarker-Ki67-positiven Zellen in humanem *engineered heart muscle* (Tag 14 nach Bestrahlung mit jeweiliger Dosis). Ki67 - Magenta; Sarkomerisches Aktinin - grün; Zellkerne - blau; Maßstab: 50 µm

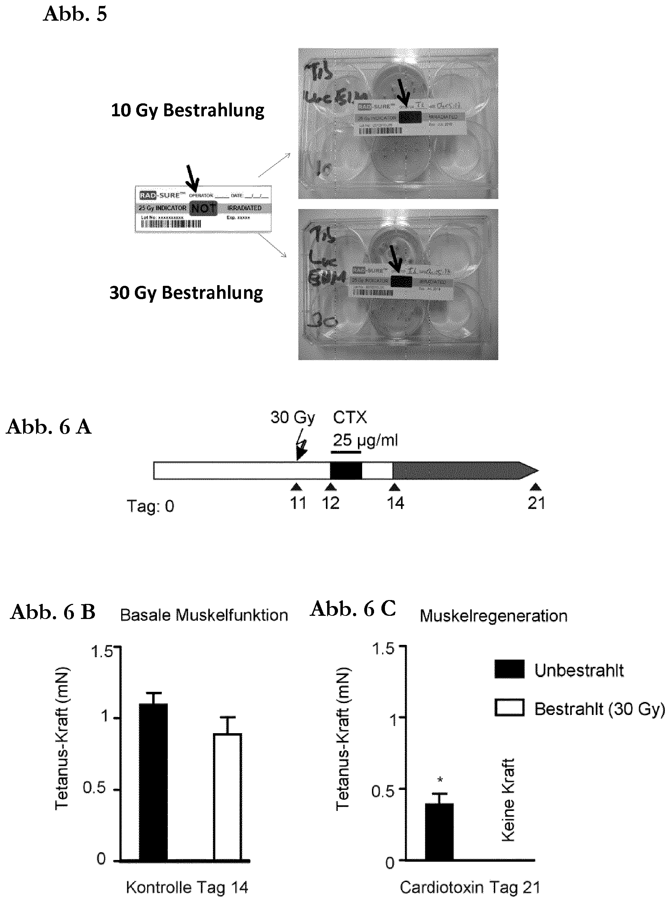

**Abbildung 5****. Validierung der Bestrahlungsdosis.** Gezeigt sind Zellkulturplatten mit EHM und 25 Gy Indikatorstreifen nach Bestrahlung mit 10 Gy (oben) und 30 Gy (unten). Der Pfeil zeigt das Indikatorfeld an, das sich nach erreichter Dosis schwarz verfärbt.

**Abbildung 6****. Bestrahlung hebt die Regenerationsfähigkeit von Skelettmuskel auf. A)** Experimentelles Protokoll: *engineered skeletal muscle* (ESM) aus Rattenzellen wurden an Tag 11 der Kultur mit 30 Gy bestrahlt und an Tag 12 der Kultur mit 25 µg/ml Cardiotoxin (CTX) für 24 Stunden geschädigt. Die Regenerationsfähigkeit wurde dann an Tag 21 mittels Messung der tetanischen Kraft überprüft. **B)** Bestrahlung hat keinen Einfluss auf ungeschädigten Muskel, die tetanische Kraft unterscheidet sich nicht zwischen unbestrahlter und bestrahlter Kontrolle. C) Bestrahlung hemmt die Regenerationsfähigkeit durch Hemmung der Proliferation und Differenzierung von Muskelstammzellen; n=4 pro Gruppe.

### Ausführliche Beschreibung

### Prinzip der Erfindung

Mit den Verfahren der vorliegenden Erfindung ist es möglich, Tumorbildung (Teratombildung) bei einem zellbasierten Produkt (TEP) durch Bestrahlung auszuschießen und gleichzeitig die biologische Funktion (z.B. Muskelkontraktion) zu erhalten. Die vorliegende Erfindung umfasst Verfahren zur mitotischen Inaktivierung durch Bestrahlung in einem prinzipiell postmitotischen Organsystem wie dem Muskel. Überraschend ist, dass die kontraktile Funktion der getesteten Herzgewebe und Skelettmuskelgewebe durch die Bestrahlung nicht beeinträchtigt wird. Dies kann damit erklärt werden, dass eine Teilungsfähigkeit der Zellen im Muskel für die biologische Funktion (Kontraktion, Krafterzeugung) nicht notwendig ist. Durch die hier gezeigten Verfahren kann erstmalig die Sicherheit eines TEP bei erhaltener biologischer Aktivität gewährleistet werden. Dadurch können die Verfahren der vorliegenden Erfindung für die klinische Anwendung von TEPs aus Stammzellen bzw. aus Zellen mit proliferativer Aktivität von entscheidender Bedeutung sein.

### Definitionen

"Erhöhung der Sicherheit" meint im Kontext der vorliegenden Erfindung eine erhöhte Sicherheit gegenüber Gefahren, die von mitotischen Zellen in einem TEP ausgehen. Die primäre Gefahr hierbei ist die Bildung von benignen Teratomen und malignen Teratocarcinomen (Tumoren ausgehend von Keimzellen). Die Verfahren der vorliegenden Erfindung beseitigen einen viele oder alle mitotische Zellen in einem TEP, wodurch die Gefahr der Tumorbildung reduziert oder minimiert wird. Dadurch ist ein TEP, das mit den Verfahren der vorliegenden Erfindung behandelt wurde, sicherer für angedachte Verwendungen wie etwa Transplantation.

*Tissue engineered product* (TEP) meint im Kontext der vorliegenden Erfindung einen künstlich hergestellten Verband von Zellen, der mindestens eine biologische Funktion eines natürlichen Gewebes aufweist. Dabei besteht der Verband von Zellen aus mehreren Zellen, die miteinander mechanisch und/oder funktional verbunden sind. Typische TEPs sind künstliche Muskelgewebe, deren biologische Funktion ist, dass sie zu Kontraktion fähig sind. Solche kontraktilen musklären TEPs umfassen beispielsweise *engineered heart muscle* (EHM) / *bioengineered heart muscle* (BHM), eine Nachbildung von Herzmuskel-Gewebe, und *engineered skeletal muscle* (ESM), eine Nachbildung von Skelettmuskelgewebe. Andere TEPs bestehen aus neuralen Zellen und weisen Eigenschaften von neuralem Gewebe auf (z.B. elektrische Signalweiterleitung zwischen Zellen, koordinierte Aktionspotentiale). Beispielhafte neurale TEPs sind *bioengineered neuronal organoids* (BENOs). Weitere TEPs bestehen aus mesenchymalen Stromazellen (z.B. Fibroblasten) und weisen Eigenschaften von Bindegewebe auf (z.B. mit Haltefunktion für die Anwendung bei Weichgewebedefekten). Diese werden auch als *engineered connective tissue* (ECT) bezeichnet. Weitere TEPs bestehen aus Hepatozyten und weisen Eigenschaften von Lebergewebe auf (z.B. Arzneimittel-Metabolisierung über Cytochrom P450-Systeme). Weitere TEPs bestehen aus Nierenzellen und weisen Eigenschaften von Nierengewebe auf (z.B. transmembranärer MDR, OAT/OCT-Transporter). Weitere TEPs bestehen aus mesenchymalen Stammzellen mit oder ohne Zusatz von Stromazellen (z.B. Fibroblasten) und weisen strukturelle und mechanische Eigenschaften von Knochengewebe bzw. der Luftröhre auf. Beispiele für TEPs sind z.B. in Tiburcy *et al.,* 2017, Wehrhan *et al.,* 2010, und zwei Publikationen von Naito *et al.,* beide von 2011, angegeben.

"Bestrahlung" meint im Kontext der vorliegenden Erfindung, dass Zellen elektromagnetischen Strahlen ausgesetzt werden. Wenn nicht anders angegeben, sind die elektromagnetischen Strahlen Röntgenstrahlen. Die Verfahren der Erfindung können aber auch mit gamma-Strahlen durchgeführt werden.

"Strahlendosis" bezieht sich auf die Energiedosis der Bestrahlung eines Materials (z.B. von Zellen). Die Energiedosis ist die über die gesamte Bestrahlungsdauer aufgenommene Energie bezogen auf die bestrahlte Masse des Materials. Die Strahlendosis / Energiedosis wird bei der Bestrahlung von Zellen üblicherweise in Gray (Gy) angegeben. Dabei entspricht ein Gray einem Joule pro Kilogramm.

*Tissue engineered muscle* (TEM) ist eine Nachbildung von Muskelgewebe. Spezielle TEMs sind EHM und ESM.

*Engineered heart muscle* (EHM) ist eine Nachbildung von Herzmuskel-Gewebe. Die Herstellung eines EHM ist zum Beispiel in Riegler *et al.,* 2015, und Tiburcy *et al.,* 2017, und WO 2015/025030 A1 beschrieben.

*Engineered skeletal muscle* (ESM) ist eine Nachbildung von Skelettmuskel-Gewebe. Die Herstellung eines ESM ist zum Beispiel im Methodenteil der Beispiele dieser Anmeldung beschrieben.

"Mitotische Zellen" oder "proliferative Zellen" (beide Ausdrücke werden hier synonym verwendet) bezieht sich auf Zellen, die Zellteilung (Mitose) durchlaufen. Ein geeigneter Marker zur Identifizierung von mitotischen Zellen ist das Protein Ki67. Dieses Protein ist in mitotischen, nicht aber in ruhenden (nicht-mitotischen) Zellen vorhanden. Neben Ki67 existieren andere Marker, die zur Identifizierung von mitotischen Zellen / Proliferation verwendet werden können, z.B. PCNA, BrdU und Cycline.

"Biologische Funktion" meint im Kontext der vorliegenden Erfindung eine Funktion eines biologischen Gewebes. TEP sind normalerweise darauf ausgelegt, mindestens eine biologische Funktion eines Gewebes nachzuahmen. Typische biologische Funktionen sind Kontraktion (Muskelgewebe), Reizweiterleitung (neurales Gewebe) und Festigkeit/Barrierefunktion (Hautgewebe).

"Im Wesentlichen erhalten" bedeutet, dass der entsprechende Wert oder die entsprechende Eigenschaft zumindest bis zu einem gewissen Grad erhalten bleibt, zum Beispiel nach einer Bestrahlung. Dieser Grad beträgt dabei mindestens 50%. Zum Beispiel beträgt eine im Wesentlichen erhaltene Kontraktionskraft nach der Bestrahlung noch mindestens 50% des Wertes vor der Bestrahlung. Der Erhaltungsgrad kann jedoch auch 60%, 70%, 80%, 90%, 95% oder 100% betragen. Generell wird ein hoher Erhaltungsgrad bevorzugt. "Im Wesentlichen erhalten" kann auch bedeuten, dass der entsprechende Wert oder die entsprechende Eigenschaft nach der Bestrahlung sogar verbessert ist, also mehr als 100% beträgt.

"Regeneration" in Zellkultur bedeutet, dass Zellen oder Zell-Verbände (wie TEPs) unter geeigneten Bedingungen kultiviert werden, also unter Bedingungen, die ein Überleben und/oder Größenwachstum der Zellen ermöglichen. Die geeigneten Bedingungen für Regeneration hängen von den Zellen des jeweiligen TEP ab und sind in der Literatur bekannt. Für EHM und ESM sind diese zum Beispiel Tiburcy *et al.,* 2017, angegeben, oder dem Methodenteil der Beispiele der vorliegenden Anmeldung zu entnehmen.

### Die Verfahren der Erfindung

Die vorliegende Erfindung umfasst zwei Hauptvarianten zur Erhöhung der Sicherheit bei einem TEP. Beide Varianten umfassen die Bestrahlung des TEP. Der Unterschied der beiden Varianten besteht darin, dass das TEP bei Variante A direkt nach der Bestrahlung verwendet werden kann, während bei Variante B nach der Bestrahlung und vor der Verwendung Regeneration in Zellkultur notwendig ist. Ein weiterer Unterschied besteht bei den geeigneten Strahlendosen für die beiden Varianten. Im Folgenden werden beide Varianten im Detail beschrieben:

### Variante A

### Wahl und Kultur des TEP

Die Verfahren der vorliegenden Erfindung können im Prinzip für jedes TEP eingesetzt werden, bei dem mitotische Zellen inaktiviert oder abgetötet werden sollen. TEPs, die hierfür geeignet sind, umfassen z.B. muskuläre TEPs (z.B. EHM, ESM, BHM), neurale TEPs (z.B. BENO), bindegewebige TEPs (z.B. ECT).

Die Herstellung von TEPs ist im Stand der Technik bekannt. Beispielsweise beschreiben Riegler *et al.,* 2015, Tiburcy *et al.,* 2017, und WO 2015/025030 A1 die Herstellung von *engineered heart muscle* (EHM).

Vor einer Bestrahlung nach einem Verfahren der vorliegenden Erfindung werden die TEP typischerweise in Zellkultur kultiviert. Die genauen Kulturbedingungen hängen von der Art des TEP ab. Sie sind in jedem Fall so gewählt, dass die Zellen des TEP überleben. Beispiele für TEP-Kulturbedingungen sind im Stand der Technik bekannt, z.B. in Tiburcy *et al.,* 2017, oder dem Methodenteil der Beispiele der vorliegenden Anmeldung zu entnehmen.

Zur Vorbereitung auf die Bestrahlung können die Kulturbedingungen geeignet geändert werden. Z.B. kann ein adhärentes TEP vom Untergrund abgelöst werden. Ein adhärentes TEP kann aber auch während der Bestrahlung die ganze Zeit adhärent bleiben. Auch kann ein TEP für die Bestrahlung in ein anderes Zellkulturgefäß überführt werden oder im alten Zellkulturgefäß verbleiben. TEPs, die an Halterungen befestigt sind (typisch für muskuläre TEPs), können zur Bestrahlung von diesen gelöst werden. Die genaue Vorbereitung hängt wiederum von der Art des jeweiligen TEP ab.

### Bestrahlung

Die Bestrahlung des TEP erfolgt mit elektromagnetischen Strahlen. Dabei können Röntgenstrahlen oder gamma-Strahlen verwendet werden. Bevorzugt sind Röntgenstrahlen. Die Bestrahlung kann mit jeder herkömmlichen Strahlenquelle erfolgen. Geeignete Strahlenquellen sind beispielsweise die Röntgenstrahlenquelle RS225A (Gulway Medical, UK) oder STS Biobeam 8000 (Gamma-Service Medical GmbH, Deutschland).

Von entscheidender Bedeutung ist die während der Bestrahlung vom TEP aufgenommene Strahlendosis. In Variante A beträgt die verwendete Strahlendosis zwischen 10 und 50 Gy. In manchen Ausführungsformen beträgt die Strahlendosis zwischen 15 und 45 Gy, zwischen 20 und 40 Gy oder zwischen 25 und 35 Gy. Beispielhafte Werte für die Strahlendosis sind 10, 15, 17, 20, 22, 25, 27, 30, 32, 35, 37, 40, 42, 45, 47 und 50 Gy. Eine besonders bevorzugte Strahlendosis ist dabei 30 Gy.

Die jeweils gewählte Strahlendosis von der Art des TEP und dem gewünschten Einsatzzweck ab. Die Strahlendosis wird so gewählt, dass die mitotischen Zellen des TEP inaktiviert oder abgetötet werden, während eine biologische Funktion des TEP erhalten bleibt. Je nach Sensitivität der Zellen des jeweiligen TEPs kommt hierbei eine genau angepasste Strahlendosis zum Einsatz. Zum Beispiel beträgt eine geeignete Strahlendosis für muskuläre TEPs (z.B. EHM oder ESM) 30 Gy. Bei dieser Strahlendosis werden die mitotischen Zellen abgetötet, während die biologische Funktion (Kontraktion) erhalten bleibt.

Die Dauer der Bestrahlung beträgt typischerweise zwischen 5 und 20 Minuten, z.B. 5, 7, 10, 13, 15, 17 oder 20 Minuten.

Zur Validierung der Strahlendosis kann z.B. ein 25 Gy-Indikatorstreifen (Rad-Sure^{™}) verwendet werden.

### Test der Funktionalität

Nach der Bestrahlung kann die Funktionalität, d.h. das Bestehen einer biologischen Funktion getestet werden. Damit wird kontrolliert, dass die biologische Funktion nicht durch die Bestrahlung verloren gegangen ist. Die Experimente zum Test der biologischen Funktion hängen von der Art des jeweiligen TEPs ab. Solche Experimente sind im Stand der Technik bekannt. Für muskuläre TEPs (z.B. EHM, ESM) kann beispielsweise die Kontraktilität (Kontraktionskraft) bestimmt werden (siehe z.B. Zimmermann *et al.,* 2000, Riegler *et al.,* 2015, Tiburcy *et al.,* 2017, und WO 2015/025030 A1). Riegler *et al.* gibt auch einen Test zur Bestimmung des Zellüberlebens an.

### Variante B

### Wahl und Kultur des TEP (siehe Variante A)

Wahl, Kultur und Strahlungsvorbereitung entsprechen dem unter "Variante A" angegebenen Abschnitt.

### Bestrahlung

Wie bei Variante A, erfolgt die Bestrahlung des TEP auch bei Variante B mit elektromagnetischen Strahlen. Dabei können Röntgenstrahlen oder gamma-Strahlen verwendet werden. Bevorzugt sind Röntgenstrahlen. Die Bestrahlung kann mit jeder herkömmlichen Strahlenquelle erfolgen. Geeignete Strahlenquellen sind beispielsweise die Röntgenstrahlenquelle RS225A (Gulway Medical, UK) oder STS Biobeam 8000 (Gamma-Service Medical GmbH, Deutschland).

Eine genau auf das zu bestrahlende TEP abgestimmte Strahlendosis ist auch bei Variante B von entscheidender Bedeutung, allerdings nicht so essentiell wie bei Variante A. In Variante B beträgt die verwendete Strahlendosis zwischen 10 und 100 Gy. In manchen Ausführungsformen beträgt die Strahlendosis zwischen 10 und 50 Gy, zwischen 20 und 40 Gy oder zwischen 25 und 35 Gy. Beispielhafte Werte für die Strahlendosis sind 10, 15, 17, 20, 22, 25, 27, 30, 32, 35, 37, 40, 42, 45, 47, 50, 55, 60, 65, 70, 75, 80, 85, 90, 95 und 100 Gy. Eine besonders bevorzugte Strahlendosis ist dabei 30 Gy.

Die jeweils gewählte Strahlendosis von der Art des TEP und dem gewünschten Einsatzzweck ab. Die Strahlendosis wird so gewählt, dass die mitotischen Zellen des TEP inaktiviert oder abgetötet werden, während eine biologische Funktion des TEP, zumindest nach einer Regenerationszeit, erhalten bleibt. Durch die Regenerationszeit muss die verwendete Strahlendosis nicht so genau für das jeweilige TEP angepasst werden wie bei Variante A. Beispielhafte geeignete Strahlendosen für muskuläre TEPs (z.B. EHM oder ESM) betragen 10, 30, 50 und 100 Gy. Bei diesen Strahlendosen werden die mitotischen Zellen abgetötet, während die biologische Funktion (Kontraktion) nach einer Regenerationszeit erhalten bleibt. Die Dauer der Bestrahlung beträgt typischerweise zwischen 5 und 20 Minuten, z.B. 5, 7, 10, 13, 15, 17 oder 20 Minuten.

Zur Validierung der Strahlendosis kann z.B. ein 25 Gy-Indikatorstreifen (Rad-Sure^{™}) verwendet werden.

### Regeneration

Die Regeneration des TEP findet unter Bedingungen statt, die ein Überleben und/oder Wachstum der Zellen des TEP ermöglichen. Die geeigneten Bedingungen für Regeneration hängen von den Zellen des jeweiligen TEP ab und sind in der Literatur bekannt. Für EHM und ESM sind diese zum Beispiel Tiburcy *et al.,* 2017, angegeben, oder dem Methodenteil der Beispiele der vorliegenden Anmeldung zu entnehmen. Die Bedingungen der Regeneration können den allgemeinen, im Stand der Technik bekannten, Kulturbedingungen des jeweiligen TEP entsprechen. Die Regeneration kann aber auch durch den Einsatz von zusätzlichen Verbindungen oder Bestandteilen gefördert werden. Solche Verbindungen und Bestandteile umfassen zum Beispiel: Wachstumsfaktoren und Cytokine (z.B. IGF-1), Chemikalien (z.B. ROCK-Inhibitor Y-27632) und Antioxidantien (z.B. Ascorbinsäure).

Die Dauer der Regeneration beträgt allgemein 1-30 Tage. In Ausnahmefällen, für sehr langsam regenerierende TEPs, können auch Regenerationsdauern von mehr als 30 (bis zu 90 Tagen) gewählt werden. Bevorzugt beträgt die Regenerationsdauer 5-25, 7-20 oder 10-15 Tage. Exemplarische Regenerationsdauern sind 5, 7, 10, 12, 14, 15, 20 oder 25 Tage.

### Kombinationen von Strahlendosen und Regenerationsdauern

Strahlendosen der Bestrahlung und anschließende Regenerationsdauern können je nach den Bedürfnissen des TEP und seiner Verwendung variiert werden. Allgemein können Strahlendosen zwischen 10 und 100 Gy mit Regenerationsdauern von 1-30 Tagen kombiniert werden. In Ausnahmefällen, für sehr langsam regenerierende TEPs, können auch Regenerationsdauern von mehr als 30 (bis zu 90 Tagen) gewählt werden.

Beispielhafte Kombinationen aus einigen Ausführungsformen sind in Tabelle 1 angegeben.

Jede dieser Kombinationen kann für jedes geeignete TEP eingesetzt werden. Besonders geeignet sind diese Kombinationen für muskuläre TEPs (z.B. EHM und ESM).

**Tabelle 1: Kombinationen von Bestrahlungsdosen und Regenerationsdauern**

| Strahlendosis [Gy] | Regenerationsdauer [Tage] | Strahlendosis [Gy] | Regenerationsdauer [Tage] | Strahlendosis [Gy] | Regenerationsdauer [Tage] |
|---|---|---|---|---|---|
| 10 | 1 | 30 | 20 | 50 | 1 |
| 10 | 3 | 30 | 22 | 50 | 3 |
| 10 | 5 | 30 | 25 | 50 | 5 |
| 10 | 10 | 30 | 27 | 50 | 10 |
| 10 | 20 | 30 | 30 | 50 | 15 |
| 20 | 1 | 35 | 1 | 50 | 20 |
| 20 | 3 | 35 | 3 | 50 | 25 |
| 20 | 5 | 35 | 5 | 50 | 30 |
| 20 | 7 | 35 | 7 | 60 | 1 |
| 20 | 10 | 35 | 10 | 60 | 3 |
| 20 | 15 | 35 | 15 | 60 | 5 |
| 20 | 20 | 35 | 20 | 60 | 10 |
| 20 | 30 | 35 | 22 | 60 | 15 |
| 25 | 1 | 35 | 25 | 60 | 20 |
| 25 | 3 | 35 | 30 | 60 | 25 |
| 25 | 5 | 40 | 1 | 60 | 30 |
| 25 | 7 | 40 | 3 | 70 | 1 |
| 25 | 10 | 40 | 5 | 70 | 5 |
| 25 | 15 | 40 | 7 | 70 | 10 |
| 25 | 20 | 40 | 10 | 70 | 20 |
| 25 | 30 | 40 | 15 | 70 | 30 |
| 30 | 1 | 40 | 17 | 80 | 1 |
| 30 | 2 | 40 | 20 | 80 | 5 |
| 30 | 3 | 40 | 22 | 80 | 10 |
| 30 | 4 | 40 | 25 | 80 | 20 |
| 30 | 5 | 40 | 30 | 80 | 30 |
| 30 | 6 | 45 | 1 | 90 | 1 |
| 30 | 7 | 45 | 3 | 90 | 10 |
| 30 | 8 | 45 | 5 | 90 | 20 |
| 30 | 9 | 45 | 7 | 90 | 30 |
| 30 | 10 | 45 | 10 | 100 | 1 |
| 30 | 12 | 45 | 15 | 100 | 5 |
| 30 | 13 | 45 | 20 | 100 | 10 |
| 30 | 15 | 45 | 25 | 100 | 20 |
| 30 | 17 | 45 | 30 | 100 | 30 |

### Test der Funktionalität (siehe Variante A)

Funktionalitätstests entsprechen dem unter "Variante A" angegebenen Abschnitt. Allerdings wird bei Variante B ein Funktionalitätstest erst nach dem Regenerationsschritt durchgeführt, nicht direkt nach der Bestrahlung.

### TEPs der vorliegenden Erfindung

Die vorliegende Erfindung umfasst auch TEPs, die mit einem Verfahren gemäß den vorhergehenden Ansprüchen behandelt worden sind, d.h. bestrahlt worden sind. Diese bestrahlten TEPs sind dadurch charakterisiert, dass sie weniger als 10% mitotische Zellen, sogar weniger als 1% mitotische Zellen oder überhaupt keine mitotischen Zellen enthalten. Der Anteil der mitotischen Zellen kann z.B. durch die Expression des Markers Ki67 bestimmt werden. Neben Ki67 existieren andere Marker und Verfahren, die zur Identifizierung von mitotischen Zellen / Proliferation verwendet werden können, z.B. PCNA, BrdU, CldU, IdU, 3H-Thymidin, Phospho-Histon H3, Cdk1, Mcm2, Cycline.

Ein TEP der vorliegenden Erfindung unterscheidet sich von TEPs, bei denen mitotische Zellen durch ein anderes Verfahren entfernt worden sind, etwa durch den Einsatz von mitotischen Inhibitoren. Die Bestrahlung ist rückstandsfrei, d.h. es lassen sich keine potential problematischen Spuren chemischer (bei Einsatz von Mitoseinhibitoren) oder genetischer Rückstände (bei genetischer Modifikation der Zellen) nachweisen. Die Bestrahlung nach den Verfahren der vorliegenden Erfindung kann durch Strahlen-induzierte Veränderungen der DNA in den Zellen des TEP nachgewiesen werden. Der Nachweis von Strahlen-induzierten Veränderungen kann durch Verfahren erfolgen, die im Stand der Technik bekannt sind, etwa durch die Bestimmung von DNA-Strangbrüchen, DNA-Basenschäden, DNA-Fragmentierung, Veränderungen an Desoxyribose-Gruppen der DNA, DNA-DNA-Vernetzungen oder DNA-Protein-Vernetzungen. Ein TEP der vorliegenden Erfindung unterscheidet sich auch von TEPs, die mit höheren Strahlendosen bestrahlt wurden, weil bei einem TEP der vorliegenden Erfindung seine biologische Funktion im Wesentlichen erhalten bleibt.

Bei den TEPs der vorliegenden Erfindung ist eine biologische Funktion (z.B. Kontraktionskraft) unverändert, verbessert oder nur wenig verschlechtert ("im Wesentlichen erhalten"), verglichen mit dem Zustand vor der Bestrahlung.

### Anwendung und Vorteile der Erfindung

### Transplantation

Eine bevorzugte Anwendung der Erfindung betrifft die Transplantation von Geweben oder Organen. TEPs, die mit den Verfahren der Erfindung behandelt wurden, sind besonders geeignet für solche Transplantationen, weil ihrer Sicherheit durch Ausschluss/Minimierung von Tumorbildung erhöht ist. Gleichzeitig besitzen die mit Verfahren der vorliegenden Erfindung behandelten TEPs noch mindestens eine biologische Funktion, die für den Patienten nach Transplantation günstig ist. Diese Funktion kann z.B. Kontraktilität bei muskulären TEPs sein.

Eine bevorzugte Ausführungsform der Erfindung ist die Verwendung von bestrahlten EHM zur Transplantation in Herzgewebe. Das EHM kann hierbei als "Herzmuskelpatch" verwendet werden. Transplantationen in Herzgewebe sind wegen der hohen Verbreitung von Herzinsuffizienz von besonderer Bedeutung. Weltweit sind mehr als 23 Millionen Patienten mit Herzmuskelschwäche registriert. Damit ist die Herzmuskelschwäche eine der häufigsten, tödlichsten und kostspieligsten Herzerkrankungen. Ab dem 40. Lebensjahr wird in 20 % der Bevölkerung eine Herzmuskelschwäche diagnostiziert. In schweren Fällen liegt die Lebenserwartung unter 12 Monaten. Heutige Wirkstoffe können die Herzinsuffizienz nur unzureichend behandeln, da sie die Erkrankung weder aufhalten noch regenerativ heilen können. Herztransplantationen sind limitiert und nicht für jede Patientengruppe geeignet. Daher sind Zelltherapien zum Herzmuskelwiederaufbau, wie die Transplantation von TEPs in Herzgewebe, ein vielversprechender Behandlungsansatz.

### Arzneimittelentwicklung

Die mit den Verfahren der Erfindung behandelten TEPs können in der Arzneimittelentwicklung verwendet werden. Bei der Entwicklung und dem Test von neuen Wirkstoffen / Arzneimitteln ist ein Modell wünschenswert, das die jeweilig physiologische oder pathologische Situation möglichst genau *in vitro* darstellt. Gerade bei der Testung von Arzneimitteln hinsichtlich Wirkung auf im Wesentlichen post-mitotische Gewebe wie Herz und Gehirn ist die Bereitstellung von TEPs ohne oder mit zumindest stark reduzierter mitotischer Aktivität von Vorteil. TEP können biologische Funktionen und die Situation in biologischen Geweben nachstellen und ermöglichen dadurch sehr aussagekräftige *in-vitro-*Experimente. Daher sind TEPs ein sehr vielversprechendes Werkzeug bei der Arzneimittelentwicklung.

### Besondere Vorteile der Erfindung

Wie oben beschrieben, birgt die Implantation von aus Stammzellen gewonnenen Zelltherapeutika (z.B. TEPs) das Risiko eines unkontrollierten Zellwachstums (sog. Teratome oder Teratocarcinome). Um dieses Problem zu lösen, verwendet die vorliegende Erfindung Bestrahlung als nicht-genetischen und leicht durchführbaren Ansatz der mitotischen Inaktivierung. Ein grundsätzlicher Vorteil der Bestrahlung (typischerweise Gamma-Strahler/Röntgenstrahlen) ist die genau Dosierbarkeit und gleichmäßige Verfügbarkeit und damit Unabhängigkeit von Diffusionsbarrieren in dicken Geweben. Bei Bestrahlung bleiben keine chemischen oder genetischen Rückstände oder Spuren wie bei anderen Verfahren, die aus regulatorischer Sicht für ein Zellprodukt höchst problematisch sind.

Gängige Bestrahlungsverfahren zerstören jedoch auch die biologischen Funktionen und biomechanischen Eigenschaften insbesondere weicher Gewebe. Die Bestrahlung gemäß der vorliegenden Erfindung ermöglicht jedoch bemerkenswerterweise eine Zell-Proliferationshemmung eines TEP (und damit Minimierung der Gefahr der Tumorbildung) unter Erhalt mindestens einer biologischen Funktion des TEP (z.B. Erhalt der biomechanische Eigenschaften = Kontraktionskraft des Muskelgewebes, siehe z.B. Abb. 2, 3 und 6).

Im Vergleich zum Stand der Technik weist die Erfindung auch die folgenden Unterschiede und Vorteile auf:
(i) statt Stammzellen wird ein Gewebe mit Bestrahlung behandelt
(ii) die Funktionsfähigkeit und Integrität des Gewebes bleibt auch nach Wochen nach Bestrahlung erhalten und
(iii) es sich hier um ein klassisches (langlebiges) Transplantat handelt und nicht um eine transiente Wirkungsweise einer Stammzellinfusion handelt.

### Beispiele

### Einleitung

Künstlich hergestellte Muskelgewebe aus humanen pluripotenten Stammzellen haben ein großes Potential als regenerative Therapien im Menschen. Zurzeit wird weltweit intensiv an der Anwendung von künstlichen Herzgeweben (*engineered heart muscle* (EHM)) bei Patienten mit Herzinsuffizienz gearbeitet. EHM aus Stammzellen zeigen bis zu 10% proliferierende Zellen unter basalen Bedingungen (siehe Abb. 1 und Abb. 4). Hier besteht grundsätzlich die Befürchtung, dass sich unter den proliferierenden Zellen tumorigene Zellen befinden könnten. Bei einer angenommenen klinischen Dosis von 1 Milliarde Zellen, bestünde demnach ein Pool an 100 Millionen potentiell tumorigenen Zellen. Durch Bestrahlung gemäß der vorliegenden Erfindung kann die Proliferation komplett oder zumindest weitestgehend (d.h. unter der Nachweisgrenze) aufgehoben werden, ohne das es zu einer Einschränkung der kontraktilen Funktion kommt (siehe Beispiele 1 und 2, Abb. 2 und 3).

Es ist auch möglich, Skelettmuskel (*engineered skeletal muscle* (ESM)) mit einer Stammzellnische herzustellen (siehe Beispiel 3). Wie *in vivo* geht die regenerative Kapazität von ESM nach Bestrahlung gemäß der vorliegenden Erfindung verloren (s. Abb. 6). Die kontraktile Funktion von ESM allerdings bleibt nach der Bestrahlung unter basalen Bedingung unverändert (s. Abbildung 6).

### Material und Methoden

Die folgenden Methoden wurden in den Beispielen 1-3 eingesetzt:

### Herstellung von EHMs

Zur Herstellung von EHMs wurden ES oder iPS-Zellen zu Herzmuskelzellen ausdifferenziert und anschließend durch geeignete Kultivierung zu EHMs verbunden. EHMs wurden wie in Tiburcy *et al.,* 2017, beschrieben hergestellt. Die einzelnen Schritte sind im Folgenden detailliert beschrieben:

### Humane ES und iPS-Zellen

Als zelluläres Ausgangsmaterial wurden die in Tiburcy *et al.,* 2017, beschriebenen pluripotenten Stammzellen (u.a. HES2 und HES3, Embryonic Stem Cell International gemäß Genehmigung durch die Zentrale Ethik-Kommission für Stammzellforschung gemäß Stammzellgesetz [StZG] - 12. Genehmigung nach dem StZG; hiPS-G1, Tiburcy *et al.,* 2017) verwendet.

### Differenzierung zu Kardiomyozyten und Isolierung

Pluripotente Stammzellen (PSCs) wurden bei einer Dichte von 5×10⁴ bis 1×10⁵ Zellen/cm² auf 1:30 Matrigel in PBS-beschichteten Platten ausplattiert und in *Knockout* DMEM, 20% *Knock-out Serum Replacement,* 2 mmol mmol/L Glutamin, 1% nicht-essentielle Aminosäuren, 100 U/mL Penicillin und 100 µg/mL Streptomycin (alles von Life Technologies) kultiviert, 1:1 gemischt mit *irradiated human foreskin fibroblast* (HFF)-konditioniertem Medium mit 10 ng/mL *fibroblast growth factor*-2 (FGF2) oder TeSR-E8 (STEMCELL Technologies). Nach einem Tag wurden die Zellen mit *Roswell Park Memorial Institute* (RPMI)-Medium gewaschen und dann für drei Tage mit RPMI, 2% B27, 200 µmol/L l-Ascorbinsäure-2-phosphat-Sesquimagnesiumsalz-Hydrate (Sigma-Aldrich), 9 ng/mL Activin A (R&D Systems), 5 ng/mL BMP4 (R&D Systems), 1 µmol/L CHIR99021 (Stemgent) und 5 ng/mL FGF-2 (Miltenyi Biotec) behandelt. Nach einem weiteren Waschschritt mit RPMI-Medium wurden die Zellen von Tag 4 bis 13 mit 5 µmol/L IWP4 (Stemgent), gefolgt von RPMI, 2% B27, 200 µmol/L l-Ascorbinsäure-2-phosphat-Sesquimagnesiumsalz-Hydrate kultiviert. Alternative Protokolle für die Herzmuskelzelldifferenzierung sind in Schuldt *et al.,* 2017, beschrieben.

### EHM-Erzeugung

Tabelle 2 (aus Tiburcy *et al.,* 2017) zeigt einen Überblick über die Protokolle, die zur Erzeugung von humanen EHMs verwendet wurden. Im Einzelnen ist die EHM Herstellung im Folgenden weiter dargestellt.

**Tabelle 2: Verschiedene Protokolle zur EHM-Erzeugung**

| **Table. Overview of EHM Protocols** | | | | |
|---|---|---|---|---|
| **Component** | | **Starting Protocol** | **Matrix Protocol** | **Serum-Free Protocol** |
| EHM reconstitution mixture | | | | |
| | Collagen rat (research grade), mg /EHM | 0.4 | | |
| | Collagen bovine (medical grade), mg lEHM | | 0.4 | 0.4 |
| | Matrigel, %, v/v | 10 | | |
| | Base medium | DMEM | DMEM | RPMI |
| | Horne serum, % | 10 | | |
| | Chick embryo extract, % | 2 | | |
| | Fetal bovine serum, % | | 20 | |
| | B27 (without insulin), % | | | 4 |

| EHM culture medium | | | | |
|---|---|---|---|---|
| | Base medium | Iscave | Iscove | Iscove* |
| | Fetal bovine serum, % | 20 | 20 | |
| | B27 (without insulin), % | | | 4 |
| | IGF-1, ng/mL | | | 100 |
| | FGF-2, ng/mL | | | 10 |
| | VEGF₁₆₅, ng/mL | | | 5 |
| | TGF-β1, ng/mL | | | 5 |
| | Nonessential amino acids, % | 1% | 1% | 1% |
| | Glutamine, mmol/L | 2 | 2 | 2 |
| | Penicillin, U/mL | 100 | 100 | 100 |
| | Streptomycin, µg/mL | 100 | 100 | 100 |
| | β-Mercaptoethanol, µmol/L | 100 | 100 | |

| | | | | |
|---|---|---|---|---|
| *Alternativ anderes Basalmedium mit ≥1,2 mmol/L Calcium. DMEM ist *Dulbecco modified* Eagle-Medium; FGF-2 ist *fibroblast growth factor*-2 (Fibroblasten-Wachstumsfaktor 2); IGF-1 ist *insulin-like growth factor* 1 (Insulinähnlicher Wachstumsfaktor 1); RPMI ist *Roswell Park Memorial Institute-Medium,* TGF-β1 ist transforming growth factor-β1 (Transformierender Wachstumsfaktor β1); VEGF165 ist *vascular endothelial growth factor* 165. | | | | |

### EHM - Grundprotokoll

In Abhängigkeit der Ausgangskultur als Zellaggregate (*embryoid bodies*) oder Einzelschichtkultur (*Monolayer*) wurden folgende Protokolle für die Herstellung von Zellsuspension verwendet:
*(1) Embryoid bodies* wurden mit Collagenase B (Roche, 1 mg/ml; H9.2), Collagenase I (Sigma-Aldrich, 2 mg/ml) und/oder Trypsin/EDTA (Life Technologies, 0,25%/1 mmol/l; HES3, HES3-ENVY, HES2, hiPS-BJ) verdaut, wie in der Literatur beschrieben (siehe auch Referenzen 1-6 aus Tiburcy *et al.,* 2017);
(2) Einzelschichten (hES2-RFP, hIPS-G1) wurden mit Accutase (Millipore), 0,0125% Trypsin (Life Technologies) und 20 µg/ml DNase (Calbiochem) für 10-15 min bei Raumtemperatur verdaut;
(3) humane Fibroblasten in Einzelschichtkultur wurden unter Verwendung von TrypLE (Life Technologies) dispergiert.

Die Fibroblasten-Kultur wurde in DMEM mit 4,5 g/l Glucose, 15% fötalem Rinderserum, 100 U/ml Penicillin und 100 µg/ml Streptomycin (alles von Life Technologies) gehalten.

Frisch dispergierte Zellen wurden unter Verwendung der *electrical currant exculsion-*Methode (CASY, Roche) gezählt, bevor mit der EHM-Konstruktion fortgefahren wurde, wobei eine Modifizierung unseres ursprünglichen EHM-Protokolls (siehe auch Referenz 7 aus Tiburcy *et al.,* 2017) zum Einsatz kam.

Kurzgefasst wurden EHMs (in einem Rekonstitutionsvolumen von typischerweise 0,5 - 8 ml) in "Gussformen" (z.B. runde Gussformen mit einem inneren/äußeren Durchmesser: 2/4 mm; Höhe: 5 mm (siehe Tiburcy *et al.,* 2014) oder flächige Gussformen von 3,5 und 3,4 cm (siehe Tiburcy *et al.,* 2017)) durch Einfüllen der EHM Rekonstruktionsmischung (Beispiele in Tabelle 2) hergestellt. Exemplarisch ist die Vorgehensweise bei Herstellung definierter Serum-freier Bedingungen aus Tiburcy *et al.,* 2017, und WO 2015/025030 A1 dargestellt: Frisch dispergierte aus PSCs abgeleitete Zellen (1×10⁴-15×10⁶ Zellen in RPMI-Medium mit 4% B27, 100 U/ml Penicillin und 100 µg/ml Streptomycin) mit oder ohne Zugabe von Fibroblasten (10-50% der Gesamtzellzahl) wurden mit pH-neutralisiertes Collagen Typ I (0,4 mg/EHM) vermischt, in Gussformen pipettiert und mit Iscove-Medium mit 4% B27 ohne Insulin, 1% nicht-essentiellen Aminosäuren, 2 mmol/l Glutamin, 300 µmol/l Ascorbinsäure, 100 ng/ml IGF1 (AF-100-11), 10 ng/ml FGF-2 (AF-100-18B), 5 ng/ml VEGF165 (AF-100-20), 5 ng/ml TGF-beta1 (AF-100-21C), 100 U/ml Penicillin und 100 µg/ml Streptomycin (siehe serumfreies Protokoll, Tabelle 2) überschichtet.

Nach einer Kondensationsphase von 3 Tagen wurden EHM aus den Gussformen auf dynamische Dehnungsvorrichtungen für die Konditionierung unter auxotonischer Kontraktion transferiert (siehe auch Referenzen 8-10 aus Tiburcy *et al.,* 2017). Das Medium wurde jeden zweiten Tag gewechselt.

### Messung der Kontraktionskraft

Die kontraktile Kraft/Funktion der EHMs wurde wie in Tiburcy *et al.,* 2017, beschrieben bestimmt. Die Messungen der Kontraktionskraft wurden unter isometrischen Bedingungen in Organ-Bädern bei 37 °C durchgeführt, in begaster (5% CO2/95% O2) Tyrode-Lösung (mit 120 NaCl, 1 MgCl2, 0,2 CaCl2, 5,4 KCl, 22,6 NaHCO3, 4,2 NaH2PO4, 5,6 Glucose, und 0,56 Ascorbat; alles in mmol/L). Die spontane Schlag-Frequenz der EHMs wurde bei 2 mmol/L Calcium nach 10 Minuten Äquilibrierung bestimmt. Die EHMs wurden mit 5 ms-Rechteck-Pulsen von 200 mA bei 1,5 bis 2 Hz elektrisch stimuliert. Die EHMs wurden in Abständen von 125 µm gedehnt, bis die maximale systolische Kraftamplitude (Kontraktionskraft) beobachtet wurde, gemäß dem Frank-Starling-Mechanismus. Untersucht wurde auch die Reaktion einzelner EHMs auf ansteigende extrazelluläre Calcium-Konzentrationen (0,2-4 mmol/L).

### Herstellung von Skelettmuskelgeweben (ESM)

Zur Herstellung von ESMs wurden Muskelzellen aus Ratten gewonnen und anschließend zu ESMs zusammengefügt.

### Isolierung und Kultur primärer Ratten-Muskelzellen

Adulte Wistar-Ratten wurden in CO2-Narkose durch zervikale Dislokation getötet. Für eine Serie von ESMs, wurde der Skelettmuskel von beiden Hinterbeinen (insgesamt 20-30 g) von einer Wistar-Ratte (6-8 Wochen alt) in Basalmedium (DMEM/F12, 5% fötales Rinderserum [FBS], 2 mmol/L Glutamin, 100 U/ml Penicillin, 100 µg/ml Streptomycin) aufgenommen (auf Eis). Bindegewebe und Sehnen wurden entfernt und der Muskel wurde zu einem feinen Brei zerkleinert. Dieser wurde für 90 min bei 37 °C mit 2,5 mg/ml Collagenase II (Biochrom) und 20 µg/ml DNAse I (Calbiochem) in Basalmedium verdaut. Die Gewebesuspension wurde anschließend gefiltert, mit Filtern absteigender Porengröße (250 µm → 100 µm → 40 µm, BD Biosciences). Die Zellsuspension wurde dann für 1 h in Basalmedium auf Zellkulturschalen ausplattiert, um die Fibroblasten-Anzahl zu reduzieren. Nicht-adhärente Zellen wurden in Proliferationsmedium (Basalmedium mit 10 ng/ml bFGF (PeproTech), 10 ng/ml EGF (PeproTech), Insulin-Transferrin-Selenite (ITS)+X (100x; Invitrogen) resuspendiert und für 5 Tage in unbeschichteten Gefäßen expandiert.

### Herstellung von ESMs

ESMs wurden unter Verwendung einer Modifikation unserer publizierten Herzmuskel-Protokolls hergestellt (siehe Naito *et al.,* 2006; Tiburcy *et al.,* 2011; Zimmermann *et al.,* 2002). Kurzgefasst wurden primäre Skelettmuskelzellen (1,5×10⁶ total Zellen/ESM), Matrix-Bestandteile (0,4 mg Rattenschwanz-Collagen, 10% Matrigel^{®}), konzentriertes Medium (2xDMEM, 20% Pferdeserum, 4% Hühnerembryo-Extract, 4 mmol/L Glutamin, 200 U/ml Penicillin, 200 µg/ml Streptomycin) gemischt und in runde "Gussformen" (innerer/äußerer Durchmesser: 4/8 mm; Höhe: 5 mm; Volumen: 450 µl) gegossen. Nach einer Stunde bei 37 °C und 5% CO2 in wassergesättigter Umgebungsluft wurde ESM-Kulturmedium (DMEM [Biochrom], 10% Pferdeserum, 2% Hühnerembryo-Extrakt, 2 mmol/L Glutamin, 100 U/ml Penicillin, 100 µg/ml Streptomycin und 1x ITS+X) zugegeben. Das Medium wurde nach 24 Stunden gewechselt, und dann jeden zweiten Tag. ESMs kondensierter innerhalb von 5 Tag und bildeten ein mechanisch robustes ringförmiges Gewebekonstrukt. Die ESMs wurden dann auf selbstgefertigte Halterungen (Dehnung auf 110% der Länge in schlaffem Zustand) transferiert und 7 weitere Tage kultiviert.

### Durchflusszytometrie

Einzelzell-Suspensionen wurden entweder lebend oder mit 70 % Ethanol oder 4% Formaldehyd (Histofix, Roth) fixiert analysiert. Für die Analyse lebender Zellen, wurde die Zellen für 10 min in *Sytox Red Dead Cell Stain* (Life Technologies, 5 nmol/L) (für den Ausschluss von toten Zellen) und in Hoechst-3342 (Life Technologies, 10 µg/ml) (für Analyse des DNA-Gehalts und Ausschluss von Dubletten) inkubiert. Zur Analyse von Ki67 wurde der Antikörper SP6 (Thermo Scientific) verwendet.

Die folgende Gating-Strategie wurde angewandt:
(1) Gating auf Zellen basierend auf *forward scatter area* (FSC-A) und *sideward scatter area* (SSC-A)
(2) Gating auf lebende Zells (*Sytox Red-*negativ)
(3) Gating auf einzelne Zellen (basierend auf der DNA-Signalbreite)

Zellen wurden mit einem LSRII SORP-Zytometer (BD Biosciences) gemessen und unter Verwendung von DIVA- oder Cyflogic-Software analysiert. Mindestens 10.000 Events wurden pro Probe analysiert.

### Immunfluorezenz

EHM-Zellen oder 2D-Einzelschichten von Kardiomyozyten wurden in 4% Formaldehyd (Histofix, Roth) fixiert. Nach 3 Waschschritten mit PBS wurden die Zellen für 2 Stunde bei Raumtemperatur oder über Nacht bei 4 °C mit den Primärantikörpern inkubiert (in PBS mit 5% Ziegenserum (Thermo Scientific), 1% Rinderserumalbumin, 0,5% Triton-X (beides von Sigma-Aldrich). Folgende Antikörper wurden verwendet: Anti-α-Aktinin (A7811, Sigma-Aldrich, Verdünnung 1:1000), anti-Ki67 (SP6, Thermo Scientific, Verdünnung 1:100). Nach mehreren Waschschritten wurden die geeigneten Sekundärantikörper (Thermo Scientific) und Hoechst-3342 (Life Technologies, 10 µg/ml) (zur Detektion von DNA im Zellkern) zugegeben, die Probe wurde für eine Stunde bei Raumtemperatur inkubiert. Die Immunfluoreszenz-Färbungen wurden mit einem konfokalen Zeiss LSM710-Mikroskop analysiert.

### ESM-Schaden

Um Skelett-Muskelschaden zu induzieren, wurde Cardiotoxin (CTX: 25 µg/ml, Latoxan) für 24 Stunden auf die ESMs angewendet.

### Bestrahlung:

Zur Bestrahlung der Muskelgewebe wurden zwei Strahlenquellen eingesetzt: Röntgenstrahlenquelle RS225A (Gulway Medical, UK) oder STS Biobeam 8000 (Gamma-Service Medical GmbH, Deutschland). Zur Validierung der Strahlendosis wurde in beiden Systemen ein 25 Gy Indikatorstreifen (Rad-Sure^{™}) verwendet.

Um Satelliten-Zellen in ESMs irreversibel zu inaktivieren, wurden ESMs von den Halterungen abgenommen und einer einzelnen Strahlendosis von 30 Gy ausgesetzt (in etwa 13 min). Die Bestrahlung erfolgte unter Verwendung der STS Biobeam 8000 (Gamma-Service Medical GmbH, Deutschland) Gamma-Strahlenquelle.

### Ergebnisse

### Beispiel 1: Einfluss von Bestrahlung auf Zellzyklusaktivtät und biologische Aktivität in menschlichem Herzmuskel

EHM aus humanen pluripotenten Stammzellen wurde zunächst mit der Standarddosis für Blutprodukte (30 Gy) zu einem frühen Zeitpunkt der Kultur (Tag 3) bestrahlt. Die Gewebe wurden dann für weitere 4 Wochen kultiviert. Mittels einer Färbung für den Zellzyklusmarker Ki67 konnte keine Zellzyklusaktivität im bestrahlten Gewebe nachgewiesen werden, während 6% der Zellen im unbestrahlten Gewebe positiv waren (**Abb. 1**). Es zeigte sich jedoch kein Unterschied in der Kontraktionskraft von unbestrahltem und bestrahltem Herzmuskel (**Abb. 2**).

### Beispiel 2: Einfluss der Strahlendosis auf die biologische Aktivität von menschlichem Herzmuskel

In einem nächsten Experiment wurde die Abhängigkeit der Strahlendosis für die Funktion von Herzgewebe untersucht. Es wurde sowohl der akute Effekt 24 Stunden nach Bestrahlung als auch die Langzeitwirkung nach 2 Wochen untersucht. Bis zu einer Bestrahlungsdosis von 100 Gy (der höchsten getesteten) ließen sich keine signifikanten Einbußen der kontraktilen Kraft akut feststellen. Auch nach zwei Wochen Regenerationszeit war bei allen getesteten Dosen außer bei 10 Gy die Kontraktionskraft vergleichbar mit der unbestrahlten Kontrolle (**Abb. 3**). Insbesondere zeigte sich auch bei den bestrahlten Geweben eine weitere Zunahme der Kraft wie unter Kontrollbedingungen, was dafür spricht, dass ein hypertrophes Wachstum der Kardiomyozyten weiterhin gegeben ist. Dies wird typischerweise bei der EHM Kultur beobachtet (Tiburcy *et al.,* 2017). Für alle Bestrahlungsdosen konnte ein vollständiger Verlust der Zellzyklusaktivität mittels Ki67-Färbung nachgewiesen werden (**Abb. 4**). Dies bestätigt die Effektivität der Bestrahlung und ist ein deutlicher Hinweis auf den Verlust der Proliferationsfähigkeit der Zellen. Zur Validierung der Bestrahlungsdosis wurde grundsätzlich ein 25 Gy Indikatorstreifen mitgeführt (**Abb. 5**).

### Beispiel 3: Einfluss von Bestrahlung auf biologische Aktivität und Regeneration in menschlichem Skelettmuskel

Ein weiterer Hinweis für die Effektivität der Bestrahlung und die Wirkung auf die Zellproliferation ergibt sich aus Experimenten mit künstlich hergestelltem Skelettmuskel (*engineered skeletal muscle* (ESM). Die Bestrahlung an sich hat im ESM-Modell wie bei den Herzmuskelexperimenten (Beispiele 1 und 2) keinen signifikanten Einfluss auf die Kontraktionskraft (**Abb. 6**). Im ESM Modell zeigt sich nach Verletzung der Gewebe, z.B. mit Cardiotoxin, eine Aktivierung und Proliferation der vorhandenen Satellitenzellen (Muskelstammzellen) und eine neue Muskelbildung mit Regeneration der kontraktilen Kraft. Diese Aktivierung und Proliferation bleibt aus, wenn der Muskel vor der Verletzung mit 30 Gy bestrahlt wurde.

### Referenzen

Burt RK, Chen YH, Verda L, Lucena C, Navale S, Johnson J, Han X, Lomasney J, Baker JM, Ngai KL, Kino A, Carr J, Kajstura J, Anversa P (2012) Mitotically inactivated embryonic stem cells can be used as an in vivo feeder layer to nurse damaged myocardium after acute myocardial infarction: a preclinical study. Circ Res. 111:1286-96
Cao F, Lin S, Xie X, Ray P, Patel M, Zhang X, Drukker M, Dylla SJ, Connolly AJ, Chen X, Weissman IL, Gambhir SS, Wu JC (2006) In vivo visualization of embryonic stem cell survival, proliferation, and migration after cardiac delivery. Circulation. 113 :1005-14.
Emami B (2013) Tolerance of Normal Tissue to Therapeutic Radiation. Reports of Radiotherapy and Oncology 1(1): 35-48
Hansen E, Knuechel R, Altmeppen J, Taeger K (1999) Blood irradiation for intraoperative autotransfusion in cancer surgery: demonstration of efficient elimination of contaminating tumor cells. Transfusion. 39:608-15.
Hudson J, Titmarsh D, Hidalgo A, Wolvetang E, Cooper-White J (2012) Primitive cardiac cells from human embryonic stem cells. Stem Cells Dev. 21:1513-1523.
Lee AS, Tang C, Cao F, Xie X, van der Bogt K, Hwang A, Connolly AJ, Robbins RC, Wu JC (2009) Effects of cell number on teratoma formation by human embryonic stem cells. Cell cycle. 8:2608-12.
Naito H, Melnychenko I, Didié M, Schneiderbanger K, Schubert P, Eschenhagen T, Zimmermann WH (2006) Optimizing Engineered Heart Tissue for Therapeutic Applications as Surrogate Heart Muscle. Circulation 114:I72-78.
Naito H, Tojo T, Kimura M, Dohi Y, Zimmermann WH, Eschenhagen T, Taniguchi S (2011) Engineering bioartificial tracheal tissue using hybrid fibroblast-mesenchymal stem cell cultures in collagen hydrogels. Interact Cardiovasc Thorac Surg. 12:156-161
Naito H, Dohi Y, Zimmermann WH, Tojo T, Takasawa S, Eschenhagen T, Taniguchi S (2011) The Effect of Mesenchymal Stem Cell Osteoblastic Differentiation on the Mechanical Properties of Engineered Bone-like Tissue. Tissue Eng Part A. 17:2321-2329.
Riegler J, Tiburcy M, Ebert A, Tzatzalos E, Raaz U, Abilez OJ, Shen Q, Kooreman NG, Neofytou E, Chen V, Wang M, Meyer T, Tsao PS, Connolly AJ, Couture LA, Gold JD, Zimmermann WH, Wu JC (2015) Human Engineered Heart Muscles Engraft and Survive Long-Term in a Rodent Myocardial Infarction Model. Circ Res 117:720-739
Schuldt AJT, Romero-Tejeda M, Burridge P (2017) Generation and Application of Human Pluripotent Stem Cell-Derived Cardiomyocytes. Erschienen in "Cardiac Regeneration, Cardiac and Vascular Biology" Herausgeber: M Ieda und WH Zimmermann, Springer Verlag
Tiburcy M, Didié M, Boy O, Christalla P, Doeker S, Naito H, Karikkineth BC, El-Armouche A, Grimm M, Nose M, Eschenhagen T, Zieseniss A, Katschinski D, Hamdani N, Linke WA, Yin X, Mayr M, Zimmermann WH (2011) Terminal Differentiation, Advanced Organotypic Maturation, and Modeling of Hypertrophie Growth in Engineered Heart Tissue. Circ Res. 109:1105-1114.
Tiburcy M, Meyer T, Soong PL, Zimmermann WH (2014) Collagen-based Engineered Heart Muscle. Methods Mol Biol. 1181:167-76.
Tiburcy M, Hudson JE, Balfanz P, Schlick S, Meyer T, Chang Liao ML, Levent E, Raad F, Zeidler S, Wingender E, Riegler J, Wang M, Gold JD, Kehat I, Wettwer E, Ravens U, Dierickx P, van Laake LW, Goumans MJ, Khadjeh S, Toischer K, Hasenfuss G, Couture LA, Unger A, Linke WA, Araki T, Neel B, Keller G, Gepstein L, Wu JC, Zimmermann WH (2017) Defined Engineered Human Myocardium With Advanced Maturation for Applications in Heart Failure Modeling and Repair. Circulation. 135:1832-1847.
Treleaven J, Gennery A, Marsh J, Norfolk D, Page L, Parker A, Saran F, Thurston J, Webb D (2011) Guidelines on the use of irradiated blood components prepared by the British Committee for Standards in Haematology blood transfusion task force. British journal of haematology. 152:35-51.
Wehrhan F, Nkenke E, Melnychenko I, Amann K, Schlegel KA, Goerlach C, Zimmermann WH, Schultze-Mosgau S (2010) Skin repair using a porcine collagen I/III membranevascularization and epithelization properties. Dermatol Surg. 36:919-930
Zimmermann WH, Fink C, Kralisch D, Remmers U, Weil J, Eschenhagen T (2000) Three-dimensional engineered heart tissue from neonatal rat cardiac myocytes. Biotechnol Bioeng 68:106-114
Zimmermann WH, Schneiderbanger K, Schubert P, Didie M, Munzel F, Heubach JF, Kostin S, Neuhuber WL, Eschenhagen T (2002) Tissue engineering of a differentiated cardiac muscle construct. Circ Res 90:223-230

## Patentansprüche

1. Ein Verfahren zur Erhöhung der Sicherheit und Verringerung der Anzahl mitotischer Zellen bei einem *tissue engineered product* (TEP), wobei das Verfahren die Bestrahlung des TEP mit einer Strahlendosis zwischen 10 und 50 Gy umfasst,
wobei die Bestrahlung mit Röntgenstrahlen oder gamma-Strahlen erfolgt.

2. Das Verfahren von Anspruch 1, wobei nach der Bestrahlung weniger als 10% mitotische Zellen im TEP vorhanden sind.

3. Das Verfahren von Anspruch 2, wobei nach der Bestrahlung weniger als 1% mitotische Zellen im TEP vorhanden sind.

4. Das Verfahren von Anspruch 1-3, wobei eine biologische Funktion des TEP nach der Bestrahlung im Wesentlichen erhalten bleibt..

5. Das Verfahren von Anspruch 1-4, wobei die Bestrahlung mit einer Strahlungsdosis von 15-45 Gy, bevorzugt 20-40 Gy, erfolgt.

6. Das Verfahren von Anspruch 5, wobei die Bestrahlung mit einer Strahlungsdosis von 25-35 Gy, bevorzugt 30 Gy, erfolgt.

7. Das Verfahren nach einem der Ansprüche 2-6, wobei mitotische Zellen durch Expression des Markers Ki67 charakterisiert sind.

8. Das Verfahren nach einem der vorhergehenden Ansprüche, wobei das TEP ein muskuläres TEP (TEM) ist.

9. Das Verfahren nach einem der vorhergehenden Ansprüche, wobei das TEP ein *engineered heart muscle* (EHM) oder ein *engineered skeletal muscle* (ESM) ist.

10. Das Verfahren von Anspruch 4, wobei die biologische Funktion Kontraktion des TEP ist.

11. Das Verfahren nach einem der Ansprüche 7-10, wobei das TEP geeignet zur Verwendung bei Transplantation und bei Arzneimittelentwicklung ist.

## Claims

1. A method for increasing safety of and decreasing the number of mitotic cells in a *tissue engineered product* (TEP),
wherein the method comprises irradiation of the TEP with a radiation dose of between 10 and 50 Gy,
wherein irradiation is performed with X-rays or gamma radiation.

2. The method according to claim 1, wherein after irradiation the number of mitotic cells in the TEP is decreased to less than 10%.

3. The method according to claim 2, wherein after irradiation the number of mitotic cells in the TEP is decreased to less than 1%.

4. The method according to claims 1-3, wherein a biological function of the TEP is essentially preserved after irradiation.

5. The method according to claims 1-4, wherein irradiation is performed at a radiation dose of 15-45 Gy, preferably of 20-40 Gy.

6. The method according to claim 5, wherein irradiation is performed at a radiation dose of 25-35 Gy, preferably of 30 Gy.

7. The method according to any of claims 2-6, wherein mitotic cells are characterized through expression of marker Ki67.

8. The method according to any of the preceding claims, wherein the TEP is a muscular TEP (TEM).

9. The method according to any of the preceding claims, wherein the TEP is an *engineered heart muscle* (EHM) or an *engineered skeletal muscle* (ESM).

10. The method according to claim 4, wherein the biological function is contraction of the TEP.

11. The method according to any of claims 7-10, wherein the TEP is suitable for use in transplantation and drug development.

## Revendications

1. Procédé pour accroître la sécurité et diminuer le nombre de cellules mitotiques pour un produit d'ingénierie tissulaire (TEP), dans lequel le procédé comporte l'irradiation du TEP avec une dose de rayons entre 10 et 50 Gy,
dans lequel l'irradiation est effectuée avec des rayons X ou gamma.

2. Procédé selon la revendication 1, dans lequel après l'irradiation, moins de 10 % de cellules mitotiques sont présentes dans le TEP.

3. Procédé selon la revendication 2, dans lequel après l'irradiation, moins de 1 % de cellules mitotiques sont présentes dans le TEP.

4. Procédé selon les revendications 1-3, dans lequel une fonction biologique du TEP reste sensiblement constante après l'irradiation.

5. Procédé selon les revendications 1-4, dans lequel l'irradiation est effectuée avec une dose de rayonnement de 15-45 Gy, de préférence de 20-40 Gy.

6. Procédé selon la revendication 5, dans lequel l'irradiation est effectuée avec une dose de rayonnement de 25-35 Gy, de préférence de 30 Gy.

7. Procédé selon l'une des revendications 2-6, dans lequel des cellules mitotiques sont **caractérisées par** expression du marqueur Ki67.

8. Procédé selon l'une des revendications précédentes, dans lequel le TEP est un TEP musculaire (TEM).

9. Procédé selon l'une des revendications précédentes, dans lequel le TEP est un muscle d'ingénierie cardiaque (EHM) ou un muscle d'ingénierie squelettique (ESM).

10. Procédé selon la revendication 4, dans lequel la fonction biologique est une contraction du TEP.

11. Procédé selon l'une des revendications 7-10, dans lequel le TEP est approprié pour l'utilisation lors de la transplantation et du développement de médicament.
